(19) Europäisches Patentamt
European Patent Office
Office européen des brevets

(11) Veröffentlichungsnummer: **0 059 372**
**A1**

(12) # EUROPÄISCHE PATENTANMELDUNG

(21) Anmeldenummer: 82101204.4

(22) Anmeldetag: 18.02.82

(51) Int. Cl.³: C 07 C 43/23
C 07 C 47/575, C 07 C 41/26
C 07 C 41/28, C 07 C 45/51

(30) Priorität: 26.02.81 DE 3107120

(43) Veröffentlichungstag der Anmeldung:
08.09.82 Patentblatt 82/36

(84) Benannte Vertragsstaaten:
BE CH DE FR GB IT LI NL

(71) Anmelder: BASF Aktiengesellschaft
Carl-Bosch-Strasse 38
D-6700 Ludwigshafen(DE)

(72) Erfinder: Barl, Manfred, Dr.
Pappelstrasse 2
D-6701 Otterstadt(DE)

(72) Erfinder: Degner, Dieter, Dr.
Kurpfalzstrasse 8
D-6701 Dannstadt-Schauernheim(DE)

(72) Erfinder: Siegel, Hardo, Dr.
Hans-Purrmann-Allee 25
D-6720 Speyer(DE)

(54) **Verfahren zur Herstellung von 4-tert.-Butoxybenzylalkohol.**

(57) Verfahren zur Herstellung von 4-Tert-Butoxybenzylalkohol, bei dem man 4-Tert-butoxybenzaldehyddialkylacetale in Gegenwart von Wasser erhitzt und das dabei erhältiche 4-Tert-butoxybenzaldehyd hydriert.

EP 0 059 372 A1

Croydon Printing Company Ltd.

Verfahren zur Herstellung von 4-tert.-Butoxybenzylalkohol

Diese Erfindung betrifft ein neues Verfahren zur Herstellung von 4-tert.-Butoxybenzylalkohol aus 4-tert.-Butoxybenzylaldehyddialkylacetalen.

4-tert.-Butoxybenzylalkohol läßt sich aus p-Nitranilin nach dem aus J. Chem. Soc. 1956, S. 2460 - 2461 bekannten Verfahren über neun Reaktionsstufen herstellen. Da 4-tert.-Butoxybenzylalkohol als ein wertvolles Zwischenprodukt neue Wege zur Synthese von Wirkstoffen ermöglicht, bestand die Aufgabe, nach einem einfacheren Verfahren zur Herstellung von 4-tert.-Butoxybenzylalkohol zu suchen.

Diese Aufgabe wurde durch das erfindungsgemäße Verfahren gelöst. Gegenstand der Erfindung ist ein Verfahren zur Herstellung von 4-tert.-Butoxybenzylalkohol, bei dem man 4-tert.-Butoxybenzaldehyddialkylacetale der Formel

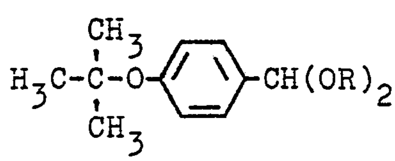

in der R eine Alkylgruppe mit 1 bis 3 C-Atomen bedeutet, in Gegenwart von Wasser erhitzt und den dabei erhältlichen 4-tert.-Butoxybenzaldehyd hydriert.

Da 4-tert.-Butoxybenzaldehyddialkylacetale auf einfache Weise durch elektrochemische Oxidation von 4-tert.-Butoxytoluol in Gegenwart eines Alkanols hergestellt werden können, eröffnet das erfindungsgemäße Verfahren einen besonders vorteilhaften Weg zur Herstellung von 4-tert.-Butoxybenzylalkohol.

Die als Ausgangsstoffe zu verwendenden 4-tert.-Butoxybenzaldehyddialkylacetale und ihre Herstellung werden in der nicht vorveröffentlichten DE-OS 29 35 398 beschrieben. Man verwendet beispielsweise das 4-tert.-Butoxybenzaldehyddimethylacetal oder das entsprechende Dialkylacetal.

Zur Verseifung wird das Diacetal in Wasser, z.B. auf Temperaturen von 40 bis 140°C, vorzugsweise 80 bis 120°C erhitzt. Man verwendet eine mindestens einmolare Menge an Wasser, bezogen auf das Diacetal. Zweckmäßigerweise verwendet man die 3- bis 20-fache molare Menge an Wasser, bezogen auf das Dialkylacetal. Besonders zweckmäßig ist es, wenn man die Dialkylacetale in dem Wasser unter Rückfluß und Rühren und ohne Abdestillieren des bei der Umsetzung entstehenden Alkohols am Sieden hält. Die Verseifung ist in der Regel nach 0,5 bis 2 Stunden beendet. Man hydrolysiert vorteilhaft in Abwesenheit von Säuren und von Lösungsmitteln. Nach der Umsetzung trennt man den 4-tert.-Butoxybenzaldehyd als organische Phase von der wäßrig-alkoholischen Phase ab. Den Aldehyd kann man dann unmittelbar oder nach seiner Destillation für die Hydrierung heranziehen.

Man hydriert den 4-tert.-Butoxybenzaldehyd z.B. in einem Lösungsmittel, wie Tetrahydrofuran, Dioxan, Ethanol, iso-Propanol oder Methanol oder auch ohne Lösungsmittel bei Temperaturen von 40 bis 140°C und Drucken von 20 bis 400 bar. Die Hydrierung wird in Gegenwart von Katalysatoren vorgenommen, die für Hydrierungen üblich sind, das sind z.B. Übergangsmetalle, wie solche der 8. Nebengruppe, von denen Eisen, Kobalt und Nickel besonders geeignet sind.

Nach den erfindungsgemäßen Verfahren erhält man den 4-tert.-Butoxybenzylalkohol glatt und in guten Ausbeuten. Das Verfahrensprodukt ist ein wertvolles Zwischenprodukt

für die Herstellung von Pharmazeutika. Beispielsweise kann es für die Herstellung des ß-Blockers der Formel

$(CH_3)_2$-CHNH-$CH_2$-CH(OH)-$CH_2$-O-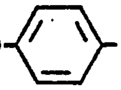-$CH_2$-CO-$NH_2$ (= Artenolol)

verwendet werden.

Beispiel

a) Herstellung von 4-tert.-Butoxybenzaldehyd

2240 g 4-tert.-Butoxybenzaldehyddimethylacetal (10 Mol) und 1680 g Wasser werden in einer Rührapparatur unter Stickstoffatmosphäre erhitzt. Man hält 2 Stunden unter Rühren und unter Rückfluß am Sieden. Die Innentemperatur fällt dabei von 98 auf 83°C. Man läßt das Reaktionsgemisch abkühlen und trennt die untere organische Phase von der überstehenden wäßrigen Methanollösung ab. Die organische Phase wird im Vakuum destilliert (Sdp. 143 bis 145°C/20 Torr). Man erhält 1709 g 4-tert.-Butoxybenzaldehyd (Ausbeute 96 %).

b) Hydrierung zum 4-tert.-Butoxybenzylalkohol

In einem 5 l-Hubrühr-Autoklaven werden 50 g Raney--Kobalt in 200 ml Methanol vorgelegt. Man preßt 20 bar Wasserstoff auf, erhitzt auf 120°C und preßt bis 150 bar Wasserstoff nach. Anschließend werden innerhalb von 3 Stunden 2136 g 4-tert.-Butoxybenzaldehyd zugepumpt, wobei man die Temperatur und den Wasserstoffdruck aufrechterhält. Der verbrauchte Wasserstoff wird stündlich ergänzt. Anschließend läßt man noch 4 Stunden nachreagieren. Die gaschromatographische Analyse des Autoklaveninhaltes zeigt einem Umsatz

von 99 % und eine Selektivität bez. 4-tert.-Butoxybenzylalkohol von 97 %. Als wichtigstes Nebenprodukt wird ca. 2 Gew.% 4-tert.-Butoxytoluol erhalten.

## Patentanspruch

Verfahren zur Herstellung von 4-tert.-Butoxybenzylalkohol, dadurch gekennzeichnet, daß man 4-tert.-Butoxybenzaldehyd-dialkylacetale der Formel

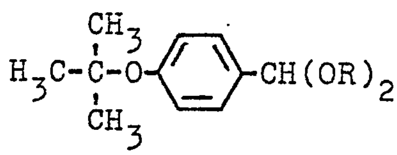

in der R eine Alkylgruppe mit 1 bis 3 C-Atomen bedeutet, in Gegenwart von Wasser erhitzt und den dabei erhältlichen 4-tert.-Butoxybenzaldehyd hydriert.

71/81 Hee/DK 25.02.1981

Hee

Europäisches Patentamt

# EUROPÄISCHER RECHERCHENBERICHT

0059372

Nummer der Anmeldung

EP 82 10 1204

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| P | EP-A-0 025 883 (BASF) * Beispiel 4 * & DE - A - 2 935 398 (Cat. D) | 1 | C 07 C 43/23<br>C 07 C 47/575<br>C 07 C 41/26<br>C 07 C 41/28<br>C 07 C 45/51 |
| A | DE-A-2 019 072 (UNIVERSAL OIL PRODUCTS) * Anspruch 1; Seite 6, Zeilen 3-7 von unten * | 1 | |
| A | DE-A-2 904 315 (BASF) * Anspruch 1 * | 1 | |
| A,D | JOURNAL OF THE CHEMICAL SOCIETY, Teil II, 1956, Seiten 2455-2462, London, G.B. G. BADDELEY et al.: "Interdependence of molecular conformation and conjugation in aromatic ethers. Part I." | 1 | |
| | | | RECHERCHIERTE SACHGEBIETE (Int. Cl. 3) |
| | | | C 07 C 41/00<br>C 07 C 45/00<br>C 07 C 47/00 |

Der vorliegende Recherchenbericht wurde für alle Patentansprüche erstellt.

| Recherchenort | Abschlußdatum der Recherche | Prüfer |
|---|---|---|
| DEN HAAG | 27-05-1982 | WRIGHT M.W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82